# EUROPEAN PATENT SPECIFICATION

(11) **EP 3 851 128 B1**
(45) Date of publication and mention of the grant of the patent: **05.03.2025**
(21) Application number: 19859397.2
(22) Date of filing: 04.06.2019
(51) Int. Cl.: A61L 15/16, A61F 13/15

(54) **DEODORIZING METHOD AND DEODORIZING ARTICLE**
DESODORIERENDES VERFAHREN UND DESODORIERENDER GEGENSTAND
PROCÉDÉ DE DÉSODORISATION ET ARTICLE DE DÉSODORISATION

(30) Priority: 11.09.2018 JP 2018170109
(43) Date of publication of application: 21.07.2021
(73) Proprietor: Kao Corporation, Chuo-ku, Tokyo 103-8210 (JP)
(72) Inventor: ISHIDA, Hirohiko, Tokyo 131-0044 (JP); TAZOE, Yuki, Tokyo 131-0044 (JP); SASAJIMA, Yukio, Haga-gun, Tochigi 321-3497 (JP)
(74) Representative: Hoffmann Eitle
(86) International application number: PCT/JP2019/022235
(87) International publication number: WO 2020/054148

(56) References cited:
- WO-A1-2017/002864
- JP-A- 2012 065 906
- JP-A- 2016 120 027
- JP-A- 2016 169 446
- JP-A- 2018 143 566
- No further relevant documents disclosed

## Description

### Technical Field

The present invention relates to a method for deodorizing a breast cancerous skin ulcer disease odor. The present invention relates to a deodorant article used for this deodorizing method.

### Background Art

It is known that a skin ulcer of breast cancer secretes a little viscous exudate accompanied with a characteristic milk-fermented odor. This odor may result in a psychological difficulty when affected individuals go out daily or obtain a job, and the QOLs of affected individuals may decrease markedly thereby.

As causative agents of a breast cancerous skin ulcer disease odor, for example, lower fatty acids and dimethyl trisulfide are known (refer to Non Patent Literature 1). A deodorant article for deodorizing a breast cancerous skin ulcer disease odor is proposed (refer to Patent Literature 1). The deodorant article described in this literature has an absorbent region and a deodorant region disposed on at least one surface side of the absorbent region. The absorbent region contains a cellulosic fiber, a water-absorbent polymer and a thermally fusion-bondable resin. The deodorant region contains a cellulosic fiber, a deodorizer and a thermally fusion-bondable resin. Although the type of odor is different, deodorant paper for a putrid urine odor is also proposed (refer to Patent Literature 2).

### Citation List

### Patent Literature

Patent Literature 1: International Publication No. WO 2017/0002864
Patent Literature 2: Japanese Patent Laid-Open No. 2016-169446

JP 2016 169446 A discloses tissue paper, containing porous particles and a pH buffering deodorant selected from among polyhydroxyamine compounds and salts thereof, that is used in an absorbent product such as a disposable diaper, and further discloses that the tissue paper can reduce the putrid odor of urine.

JP 2012 065906 A teaches that the malodorous substance associated with breast cancer is dimethyl trisulfide.

### Non Patent Literature

Non Patent Literature 1: Mika Shirasu et al., Biosci. Biotechnol. Biochem., vol.73, No.9, pp.2117-2120:2009

### Summary of Invention

The present invention provides a method for deodorizing a breast cancerous skin ulcer disease odor emitted from a breast cancerous skin ulcer site and including mainly acetoin using a deodorant article including a sheet-like covering.

The sheet-like covering contains a polyhydroxyamine compound or a salt thereof and porous particles, a wound contact surface in the sheet-like covering includes an air-through nonwoven fabric, and the sheet-like covering has a value of a ratio of an area S (cm²) to a thickness T (cm), S/T, of 300 or more and 3000 or less.

The present invention provides a deodorant article including a sheet-like covering, wherein the deodorant article is disposed at a breast cancerous skin ulcer site and used for deodorizing a breast cancerous skin ulcer disease odor emitted from the site and including mainly acetoin.

The sheet-like covering contains a polyhydroxyamine compound or a salt thereof and porous particles, a wound contact surface in the sheet-like covering includes an air-through nonwoven fabric, and the sheet-like covering has a value of a ratio of an area S (cm²) to a thickness T (cm), S/T, of 300 or more and 3000 or less.

Further, the present invention provides use of a sheet-like covering for deodorizing a breast cancerous skin ulcer disease odor emitted from a breast cancerous skin ulcer site and including mainly acetoin, wherein the sheet-like covering contains a polyhydroxyamine compound or a salt thereof and porous particles, a wound contact surface in the sheet-like covering includes an air-through nonwoven fabric, and the sheet-like covering has a value of a ratio of an area S (cm²) to a thickness T (cm), S/T, of 300 or more and 3000 or less.

### Brief Description of Drawings

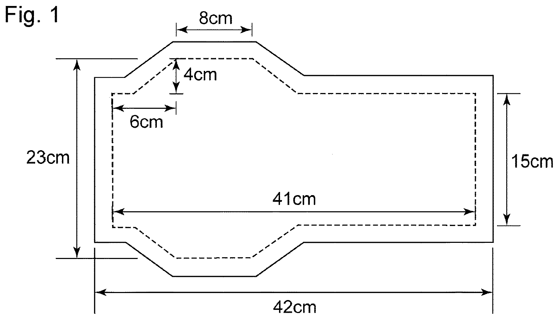

[Fig. 1] Fig. 1 is a schematic top view of a deodorant article manufactured in Examples.

### Description of Embodiments

A deodorant effect on a breast cancerous skin ulcer disease odor of remained insufficient by even the technique described in Patent Literature 1, and had room for improvement. Therefore, the present invention relates to a deodorizing method and a deodorant article which enables solving a disadvantage which the above-mentioned conventional technology has.

The present invention will be described hereinafter based on the preferred embodiments. The present invention relates to the deodorization of a breast cancerous skin ulcer disease odor. In the present invention, offensive odor components are made to reach a deodorant part in a deodorant article effectively by disposing the deodorant article at a breast cancerous skin ulcer site, and a breast cancerous skin ulcer disease odor emitted from the site is thus deodorized. As mentioned above, although the conventional techniques as described above were proposed to deodorize a breast cancerous skin ulcer disease odor, an enough deodorant effect could not be obtained by those techniques. The present inventors have examined reasons therefor in detail in view of offensive odor components emitted from a breast cancerous skin ulcer site and a deodorizing method thereof. Consequently, the following facts (1) to (3) have become clear.
(1) Since an exudate emitted from a breast cancerous skin ulcer site has high viscosity, and is discharged from an affected part having a large area at a very low speed (around 0.12 g/minute; around 1/3000 times the speed of urine) unlike urine or menstrual blood, the exudate easily consolidates on the surface of the site. Therefore, even when a deodorant article is disposed at the site, offensive odor components hardly reach a deodorant part in the deodorant article, and an enough deodorant effect is not obtained.
(2) Acetoin has been found, which was not known in the past as an offensive odor component of a breast cancerous skin ulcer disease odor and is a substance on which a deodorant effect was insufficient. It has been found that the deodorization of especially acetoin among the many causative agents of a breast cancerous skin ulcer disease odor is effective in the deodorization of the breast cancerous skin ulcer disease odor.
(3) It is known that a breast cancerous skin ulcer disease odor is emitted from many parts such as the top of the mamma and the axillary area in which lymph glands exist. When a plurality of simply rectangular deodorant articles is used and disposed at parts, the deodorant articles fall off easily. Although it is also considered that the deodorant articles are fixed on the skin using adhesive tape, the use of adhesive tape involves pain at the time of the peeling thereof, and the use thereof is therefore avoided in many cases.

In the present invention, suitably, (i) a sheet-like covering having a specific structure is used as a deodorant article, (ii) the thickness and length of the sheet-like covering are balanced appropriately, (iii) an air-through nonwoven fabric is used as especially a topsheet, and (iv) a specific deodorizer having a deodorant effect on acetoin is adopted, wherein the present inventors have first found that acetoin is a substance emitted from a breast cancerous skin ulcer site, and that the deodorization of especially this substance among many causative agents of a breast cancerous skin ulcer disease odor is effective in the reduction and deodorization of the breast cancerous skin ulcer disease odor. In this way, offensive odor components emitted from a breast cancerous skin ulcer site would be made to reach the inside of the sheet-like covering rapidly, and the deodorant article worn on the body of a wearer is also hardly dislocated besides enabling the deodorant article to exhibit an enough deodorant effect. Consequently, the range of the wearer's daily activity is widened, and improvement in QOL can be expected.

The present invention will be described in detail hereinafter based on the above-mentioned facts. First, the air-through nonwoven fabric used as the topsheet in the above-mentioned sheet-like covering will be described. The air-through nonwoven fabric is obtained by blowing hot air on a web containing thermally fusion-bondable fibers by a penetration process and fusing intersection points of thermally fusion-bondable fibers. The air-through nonwoven fabric has a low density and is bulky due to the manufacturing method thereof. Consequently, the air-through nonwoven fabric has the characteristics of a long distance between constituent fibers and high air permeability. The air-through nonwoven fabric also has the characteristics of high cushioning properties and a good feel to the skin. The air-through nonwoven fabric is used as a wound contact surface in the sheet-like covering in the present invention by making the most of these characteristics which the air-through nonwoven fabric has. Consequently, even though an exudate emitted from a wound contact surface, namely a breast cancerous skin ulcer site is discharged at a very low speed, and consolidates on the surface of the site, offensive odor components emitted from the wound contact surface side can rapidly reach the inside of the sheet-like covering through the air-through nonwoven fabric, and the rapid deodorization is enabled.

The air-through nonwoven fabric having a density of 5.0 × 10⁻¹ g/cm³ or less, especially 3.0 × 10⁻¹g/cm³ or less, and particularly 1.0 × 10⁻¹ g/cm³ or less is preferably used in view of making the above-mentioned advantage resulting from the use of the air-through nonwoven fabric still more remarkable. A lower density of the air-through nonwoven fabric is more preferable in view of enhancing the air permeability. However, when the density is excessively low, the strength of the air-through nonwoven fabric may decrease greatly. Then, it is preferable that the density of the air-through nonwoven fabric be 1.0 × 10⁻³ g/cm³ or more, especially 5.0 × 10⁻³ g/cm³ or more, particularly 1.0 × 10⁻² g/cm³ or more. The density of the air-through nonwoven fabric is calculated by dividing the basis weight of the air-through nonwoven fabric by the thickness. The thickness of an air-through nonwoven fabric is measured under a load of 2.5 g/cm². For example, a non-contact laser displacement meter or the like can be used for measurement.

The thickness of the air-through nonwoven fabric is preferably 0.2 mm or more, more preferably 0.25 mm or more, and even more preferably 0.3 mm or more under a load of 2.5 g/cm² in view of the above-mentioned relationship between the thickness and the density and a good touch. The thickness is preferably 4 mm or less, more preferably 3 mm or less, and even more preferably 2 mm or less.

The basis weight of the air-through nonwoven fabric is preferably 10 g/m² or more, more preferably 12 g/m² or more, and even more preferably 14 g/m² or more from the same viewpoints. The basis weight is preferably 60 g/m² or less, more preferably 50 g/m² or less, and even more preferably 40 g/m² or less.

The fibers constituting the air-through nonwoven fabric preferably contains at least thermally fusion-bondable fibers. Examples of the thermally fusion-bondable fibers include core-sheath conjugate fibers having a low melting point resin as a sheath component and a high melting point resin as a core component and side-by-side conjugate fibers in which low melting point resin parts and high melting point resin parts are arranged in a line so as to extend in one direction. Examples of the low melting point resin include polyethylene and low melting point polypropylene. Examples of the high melting point resin include polypropylene and polyethylene terephthalate.

In the deodorant article including the sheet-like covering, an air-through nonwoven fabric may be used for the whole region of the topsheet, or an air-through nonwoven fabric may be used only for the wound contact surface in the topsheet.

The sheet-like covering, in which the above-mentioned air-through nonwoven fabric is used, has a liquid-permeable topsheet, a liquid-impermeable or sparingly-liquid-permeable backsheet, and a liquid-retentive absorbent core disposed therebetween.

In the plane view, the sheet-like covering generally has a longitudinal direction and a width direction perpendicular thereto, and has a shape which is long in the longitudinal direction. Although the sheet-like covering is generally rectangular in the plane view thereof, the sheet-like covering is not limited to this shape.

As the backsheet in the sheet-like covering, a film made of a thermoplastic resin or a film obtained by pasting the film and a fiber sheet such as a nonwoven fabric together can be used. This film may be liquid-impermeable and steam-permeable. A nonwoven fabric having a multilayer structure and formed by laminating a plurality of types of nonwoven fabrics can also be used as the sparingly-liquid-permeable backsheet. Examples of such a nonwoven fabric having a multilayer structure include a spunbond-meltblown-spunbond (SMS) nonwoven fabric and a spunbond-meltblown (SM) nonwoven fabric.

The absorbent core is disposed between the backsheet and the topsheet in the sheet-like covering. The absorbent core is sealed in a space defined with the backsheet and the topsheet. Examples of the absorbent core include a stack of fibers of water-absorbent fiber materials and a stack of fibers containing water-absorbent fiber materials and water-absorbent polymers. Alternatively, a layer containing only a water-absorbent polymer and a water-absorbent sheet in a form in which a water-absorbent polymer is immobilized on a fiber sheet can also be used as the absorbent core. The form of the absorbent core used may be selected appropriately depending on a specific shape, thickness and the like of the sheet-like covering.

Various hydrogel materials can be used as the above-mentioned water-absorbent polymer. For example, a crosslinked product of a polymer or a copolymer of acrylic acid or an alkali metal acrylate; crosslinked products of polyacrylic acid, a salt thereof, and a polyacrylate graft polymer; a crosslinked product of starch or carboxymethylated cellulose; a crosslinked product of a hydrolyzate of a starch-acrylate graft copolymer; a crosslinked product of a vinyl alcohol-acrylate copolymer; a crosslinked product of maleic anhydride-grafted polyvinyl alcohol; a crosslinked isobutylene-maleic anhydride copolymer; a saponified product of a vinyl acetate-acrylic acid ester copolymer; or the like can be used.

At least one of the surface opposite to the topsheet and the surface opposite to the backsheet of the absorbent core may be covered with the core-wrap sheet having liquid permeability. For example, two core-wrap sheets of the same type or different types are used, and the core-wrap sheets can be disposed on the surface opposite to the topsheet and the surface opposite to the backsheet in the absorbent core. Alternatively, one core-wrap sheet is used, and the core-wrap sheet can be wound around the surface of the absorbent core and disposed on the surface opposite to the topsheet and the surface opposite to the backsheet in the absorbent core. Examples of the core-wrap sheet include nonwoven fabrics and tissue paper (thin paper). A deodorant effect can also be enhanced by winding the core-wrap sheet around all the surfaces of the absorbent core and further adding one or more core-wrap sheets to the surface opposite to the topsheet or one or more core-wrap sheets to the surface opposite to the backsheet.

The present inventors have found that when the ratio of the area to the thickness of a sheet-like covering is not in an appropriate range, for example, when a too thin sheet-like covering is used, an exudate consolidates in a large range of the surface of a sheet-like covering since the amount of an exudate absorbed in the direction perpendicular to the surface of the sheet-like covering is little, and that offensive odor components thus hardly reach the deodorant part in the deodorant article to thereby fail to obtain an enough deodorant effect. The present inventors have found that on the other hand, when a too thick sheet-like covering is used, a large amount of exudate is absorbed in a direction perpendicular to the surface of the sheet-like covering to result in increase in the amount of water, and that the nucleophilic chemical reaction of acetoin and a polyhydroxyamine compound is therefore delayed. The present inventors have also found that since the mass of the sheet-like covering increases locally with this, the worn sheet-like covering is dislocated from an appropriate position, and that an enough deodorant effect is thus not obtained. The present inventors have earnestly examined to solve these troubles. Consequently, it has become clear that adjusting the ratio of the area to the thickness of the sheet-like covering to an appropriate range brings about moderate water diffusion effect, which enables promoting the nucleophilic chemical reaction of a polyhydroxyamine compound with acetoin by a while preventing the consolidation of an exudate in a large range, thereby obtaining an enough deodorant effect and preventing the dislocation of the sheet-like covering effectively. Specifically, it has become clear that when the thickness and the area of the sheet-like covering are designated as T (cm) and S (cm²), respectively, it is effective to adjust the value of the ratio of the area S to the thickness T, S/T, to 300 or more, especially 400 or more, further 500 or more, further 800 or more, particularly 1000 or more. It has become clear that it is effective to adjust the value of the S/T to 3000 or less, especially 2500 or less, further 2000 or less, particularly 1500 or less. The value of the S/T is preferably adjusted to 300 or more and 3000 or less, especially 400 or more and 2500 or less, further 500 or more and 2500 or less, further 800 or more and 2000 or less, particularly 1000 or more and 1500 or less.

The thickness of the sheet-like covering T is preferably 0.2 cm or more, more preferably 0.3 cm or more, and even more preferably 0.4 cm or more in view of obtaining an enough deodorant effect and still more effectively preventing the dislocation of the worn sheet-like covering. The thickness T is preferably 1.5 cm or less, more preferably 1.2 cm or less, and even more preferably 1.0 cm or less. The thickness T is preferably especially 0.2 cm or more and 1.5 cm or less, more preferably 0.3 cm or more and 1.2 cm or less, and even more preferably 0.4 cm or more and 1.0 cm or less.

Measurement is performed using a constant-pressure thickness gauge (in accordance with JIS K6402, manufactured by TECLOCK Co., Ltd., PG-11), the average value of the values of five portions which are the thickest structurally is calculated, and the nearest tenth cm of the average value is rounded off to calculate the thickness of the sheet-like covering. Measurement is performed at positions where the absorbent core exists in the sheet-like covering.

The area of the sheet-like covering S is preferably 200 cm² or more, more preferably 250 cm² or more, more preferably 400 cm² or more, and even more preferably 600 cm² or more from the same viewpoints as the thickness T. The area S is preferably 2000 cm² or less, more preferably 1500 cm² or less, more preferably 1200 cm² or less, even more preferably 1000 cm² or less, and further even more preferably 900 cm² or less. Especially, the area S is preferably 200 cm² or more and 2000 cm² or less, more preferably 250 cm² or more and 1500 cm² or less, even more preferably 400 cm² or more and 1200 cm² or less, further even more preferably 600 cm² or more and 1000 cm² or less, and further even more preferably 600 cm² or more and 900 cm² or less. The area of the sheet-like covering is measured using a tape measure or the like and determined by calculation. A region in which the absorbent core exists in the sheet-like covering is measured as a target. Therefore, the periphery in the sheet-like covering, namely a region in which only the topsheet and/or the backsheet exist is not included in an area to be measured.

The sheet-like covering preferably has a shape which is long in one direction in view of enabling covering a large range from the top of the mamma of a wearer to the axillary area. Especially, the length in the longitudinal direction of the sheet-like covering L is preferably 15 cm or more, more preferably 20 cm or more, more preferably 25 cm or more, and even more preferably 35 cm or more. The length L is preferably 60 cm or less, more preferably 55 cm or less, and even more preferably 50 cm or less. Especially, the length L is preferably 15 cm or more and 60 cm or less, more preferably 20 cm or more and 55 cm or less, more preferably 25 cm or more and 55 cm or less, and even more preferably 35 cm or more and 50 cm or less. The length of the sheet-like covering L is measured using a tape measure or the like. A region in which the absorbent core exists in the sheet-like covering is measured as a target.

When the sheet-like covering has a shape which is long in one direction, the length in a direction perpendicular to the longitudinal direction of the sheet-like covering, namely the width W of a sheet-like covering, may be constant, or may be different depending on the portion. For example, the width in a specific position in the longitudinal direction of the sheet-like covering may be large, or may be small as compared with other positions. When the width W of the sheet-like covering is constant, the width W is preferably 13 cm or more, more preferably 15 cm or more, and even more preferably 20 cm or more in view of enabling covering a breast cancerous skin ulcer site fully. The width W is preferably 30 cm or less, more preferably 28 cm or less, and even more preferably 26 cm or less. The width W is especially preferably 13 cm or more and 30 cm or less, more preferably 15 cm or more and 28 cm or less, and even more preferably 20 cm or more and 26 cm or less. When the width of the sheet-like covering is different depending on the portion, the width at a part having a minimum width is preferably in the above-mentioned range. The width W of the sheet-like covering is measured using a tape measure or the like. A region in which the absorbent core exists in the sheet-like covering is measured as a target.

The sheet-like covering contains the deodorizer for a breast cancerous skin ulcer disease odor emitted from a breast cancerous skin ulcer site. As mentioned above, the present inventors have searched for a causative agent of a breast cancerous skin ulcer disease odor and consequently first found that acetoin, which is a causative agent not known to date, is emitted from a breast cancerous skin ulcer site. The present inventors have examined and consequently revealed that acetoin is contained in a breast cancerous skin ulcer disease odor, and is a causative agent which is included mainly in the ulcer disease odor, and that a breast cancerous skin ulcer disease odor can be reduced by deodorizing acetoin.

The standard nomenclature of acetoin is 3-hydroxy-2-butanone, and acetoin is a compound having the structure of an a-hydroxy ketone. The present inventors have searched for a substance which also enables deodorizing acetoin in addition to offensive odor components of a breast cancerous skin ulcer disease odor known conventionally, for example, lower fatty acids and dimethyl trisulfide, and consequently found that incorporating the combination of a polyhydroxyamine compound or a salt thereof with porous particles into a sheet-like covering enables deodorization by both aspects of chemical deodorization and physical deodorization, and that a higher deodorant effect is thus obtained than by conventional deodorant articles.

Regardless of what part of the sheet-like covering the polyhydroxyamine compound or the salt thereof and the porous particles are contained in, the polyhydroxyamine compound or the salt thereof and the porous particles exhibit a deodorant effect. Especially when one or more of the polyhydroxyamine compound or the salt thereof and the porous particles are contained in a part other than the wound contact surface in the topsheet in the sheet-like covering, a deodorant effect is still highly exhibited, which is preferable. For example, one or more of the polyhydroxyamine compound or the salt thereof and the porous particles can be incorporated into the absorbent core or incorporated into the surface opposite to the absorbent core in the topsheet.

Especially when one or more of the polyhydroxyamine compound or the salt thereof and the porous particles are contained between the topsheet and the absorbent core or between the backsheet and the absorbent core, a deodorant effect is still highly exhibited, which is preferable. For example, a liquid-permeable sublayer sheet can be disposed between the topsheet and the absorbent core, and one or more of the polyhydroxyamine compound or the salt thereof and the porous particles can be incorporated into the sublayer sheet.

One or more of the polyhydroxyamine compound or the salt thereof and the porous particles are even more preferably incorporated into the core-wrap sheet covering the absorbent core. In this way, a breast cancerous skin ulcer disease odor including mainly acetoin can be deodorized very effectively. Advantageously, core-wrap sheets are disposed especially on the surface opposite to the topsheet and the surface opposite to the backsheet in the absorbent core, and either or both of the polyhydroxyamine compound or the salt thereof and the porous particles are incorporated into the core-wrap sheets.

For example, the deodorant article can be configured in the following manner: the polyhydroxyamine compound or the salt thereof is incorporated into the core-wrap sheet disposed on the surface opposite to the topsheet in the absorbent core without incorporating the porous particles thereinto, and simultaneously, the porous particles are incorporated into the core-wrap sheet disposed on the surface opposite to the backsheet in the absorbent core without incorporating the polyhydroxyamine compound or the salt thereof thereinto. In this way, pores of porous particles can be prevented from being blocked with an exudate emitted from a breast cancerous skin ulcer site, and both chemical deodorization and physical deodorization can be performed successfully.

Alternatively, the deodorant article can be configured in the following manner: the polyhydroxyamine compound or the salt thereof is incorporated into the core-wrap sheet disposed on the surface opposite to the topsheet in the absorbent core without incorporating the porous particles thereinto, and simultaneously, the polyhydroxyamine compound or the salt thereof and the porous particles are incorporated into the core-wrap sheet disposed on the surface opposite to the backsheet in the absorbent core. This way of disposition also enables performing both chemical deodorization and physical deodorization successfully.

To incorporate the polyhydroxyamine compound or the salt thereof and porous particles into the sheet-like covering may be achieved by, for example, adopting a step of spraying an aqueous dispersion containing the polyhydroxyamine compound or the salt thereof or the porous particles on any fiber material for forming the sheet-like covering, for example, the above-mentioned core-wrap sheet, or impregnating any fiber material with the aqueous dispersion to adhere the polyhydroxyamine compound or the salt thereof or porous particles thereto. A target deodorant article can be thus manufactured.

To deodorize a breast cancerous skin ulcer disease odor including mainly acetoin effectively, the polyhydroxyamine compound or the salt thereof is contained in a single sheet-like covering in an amount of preferably 0.0005 g or more, more preferably 0.001 g or more, even more preferably 0.005g or more in terms of the total amount thereof. The polyhydroxyamine compound or the salt thereof is contained in a single sheet-like covering in an amount of preferably 0.05 g or less, more preferably 0.03 g or less, even more preferably 0.02 g or less in terms of the total amount thereof. Especially, the amount of the polyhydroxyamine compound or the salt thereof contained in a sheet-like covering is preferably 0.0005 g/sheet or more and 0.05 g/sheet or less, more preferably 0.001 g/sheet or more and 0.03 g/sheet or less, and even more preferably 0.005 g/sheet or more and 0.02 g/sheet or less in terms of the total amount thereof.

The polyhydroxyamine compound or the salt thereof is contained in the sheet-like covering in an amount of preferably 0.01 g/m² or more, more preferably 0.05 g/m² or more in terms of the total amount thereof from the same viewpoint as the above. The polyhydroxyamine compound or the salt thereof is contained in the sheet-like covering in an amount of preferably 3.0 g/m² or less, more preferably 1.0 g/m² or less in terms of the total amount thereof. The polyhydroxyamine compound or the salt thereof is contained in the sheet-like covering in an amount of preferably 0.01 g/m² or more and 3.0 g/m² or less, more preferably 0.05 g/m² or more and 1.0 g/m² or less in terms of the total amount thereof. The area in this case is the area of the absorbent core.

The porous particles are contained in the sheet-like covering in an amount of preferably 0.005 g/m² or more, more preferably 0.01 g/m² or more, even more preferably 0.05 g/m² or more. The porous particles are contained in an amount of preferably 5.0 g/m² or less, more preferably 3.0 g/m² or less, even more preferably 2.0 g/m² or less. Especially, the content of the porous particles in the sheet-like covering is preferably 0.005 g/m² or more and 5.0 g/m² or less, more preferably 0.01 g/m² or more and 3.0 g/m² or less, and even more preferably 0.05 g/m² or more and 2.0 g/m² or less. The area in this case is the area of the absorbent core.

For a polyhydroxyamine compound which can be used in the present invention, a polyhydroxyamine compound used preferably is represented by the following formula (1).

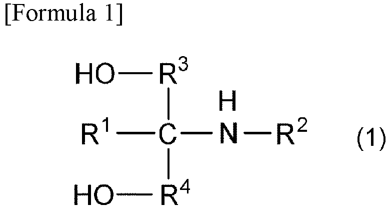

In formula (1), R¹ represents a hydrogen atom, an alkyl group having 1 to 5 carbon atoms or a hydroxyalkyl group having 1 to 5 carbon atoms. The alkyl group having 1 to 5 carbon atoms may be any of a linear chain and a branched chain, and examples of the alkyl group include a methyl group, an ethyl group, an n-propyl group, an isopropyl group, various butyl groups, and various pentyl groups. Examples of the hydroxyalkyl group having 1 to 5 carbon atoms include a hydroxymethyl group, a 2-hydroxyethyl group, a 2-hydroxypropyl group, a 3-hydroxypropyl group, a 2-hydroxybutyl group, a 3-hydroxybutyl group, and a 4-hydroxybutyl group. The R¹ is preferably a hydrogen atom, a methyl group, an ethyl group, a hydroxymethyl group, or 2-hydroxyethyl group; and more preferably a hydrogen atom, a hydroxymethyl group, or a 2-hydroxyethyl group, in view of large capacity to chemically deodorize acetoin.

R² represents a hydrogen atom, an alkyl group having 1 to 6 carbon atoms, or a hydroxyalkyl group having 1 to 5 carbon atoms. The alkyl group having 1 to 6 carbon atoms may be any of a linear chain, a branched chain and a cyclic chain. Specific examples of the alkyl group include a methyl group, an ethyl group, an n-propyl group, an isopropyl group, various butyl groups, various pentyl groups, various hexyl groups, a cyclopentyl group, and a cyclohexyl group. Examples of the hydroxyalkyl group having 1 to 5 carbon atoms include the above. The R² is preferably a hydrogen atom, an alkyl group having 1 to 3 carbon atoms, or a hydroxyethyl group; and more preferably a hydrogen atom, in view of high capability to chemically deodorize acetoin.

R³ and R⁴ each represents an alkanediyl group having 1 to 5 carbon atoms. R³ and R⁴ may be the same or different. The alkanediyl group having 1 to 5 carbon atoms is preferably a methylene group, an ethylene group, a trimethylene group, a propane-1,2-diyl group, or a tetramethylene group; and more preferably a methylene group.

Specific examples of the polyhydroxyamine compound which can be used in the present invention include 2-amino-1,3-propanediol, 2-amino-2-methyl-1,3-propanediol, 2-amino-2-ethyl-1,3-propanediol, 2-amino-2-hydroxymethyl-1,3-propanediol, 2-amino-2-hydroxyethyl-1,3-propanediol, 4-amino-4-hydroxypropyl-1,7-heptanediol, 2-(N-ethyl)amino-1,3-propanediol, 2-(N-ethyl)amino-2-hydroxymethyl-1,3-propanediol, 2-(N-decyl)amino-1,3-propanediol, 2-(N-decyl)amino-2-hydroxymethyl-1,3-propanediol, and salts thereof. Examples of the salts of these compounds include alkali metal salts such as sodium salts and potassium salts. These polyhydroxyamine compounds or salts thereof can be used singly or in combination of two or more. Among these, one or more selected from 2-amino-1,3-propanediol, 2-amino-2-methyl-1,3-propanediol, 2-amino-2-ethyl-1,3-propanediol, 2-amino-2-hydroxymethyl-1,3-propanediol, 2-amino-2-hydroxyethyl-1,3-propanediol, and salts thereof are more preferable in view of large capacity to chemically deodorize acetoin. Especially, 2-amino-2-hydroxymethyl-1,3-propanediol or a salt thereof, namely tris(hydroxymethyl)aminomethane or a salt thereof, is preferable in view of excellent capacity to deodorize acetoin and low volatility which results in its own little smell.

Regarding the deodorization of offensive odor substances, it is known that the neutralizing effect of a polyhydroxyamine compound is used for deodorizing urine odor. For example, in Patent Literature 2, it is disclosed that thin paper in which tris(hydroxymethyl)aminomethane is incorporated into a cellulosic fiber sheet base material is used as a sheet which covers an absorbent core in an absorbent article. In the same literature, deodorization is performed by neutralizing causative agents of a urine odor using a pH-buffering effect of a polyhydroxyamine compound. In contrast, acetoin, which is one of the causative agents of a breast cancerous skin ulcer disease odor, is chemically deodorized in the present invention by reacting acetoin with a polyhydroxyamine compound using nucleophilicity of a polyhydroxyamine compound. This chemical reaction is promoted by setting the ratio of the area to the thickness of a sheet-like covering in an appropriate range and thus producing a moderate water diffusion effect while the consolidation of exudate is prevented in a larger range. Consequently, an enough deodorant effect is obtained.

The porous particles used in combination with the polyhydroxyamine compound or the salt thereof enable adsorbing or including causative agents of a breast cancerous skin ulcer disease odor including mainly acetoin in pores thereof. Porous particles mean particulates with many pores, and the material thereof may be inorganic or organic. The average pore size of the porous particles is preferably 0.1 nm or more in view of efficiently adsorbing or including causative agents of a breast cancerous skin ulcer disease odor including mainly acetoin. The shape of porous particles may be any of a spherical shape; a rod shape and a needle shape having aspect ratios; an indefinite shape such as a pulverized shape; and the like.

Examples of the porous particles include synthetic porous polymers such as acrylic acid-based polymers such as a porous methacrylic acid polymer and a porous acrylic acid polymer, aromatic polymers such as a porous divinylbenzene polymer and a porous pyridine copolymer, and copolymers thereof; natural porous polymers such as chitin and chitosan; inorganic porous substances such as activated carbon, zinc oxide, aluminum oxide, silicon dioxide (silica gel), aluminosilicates, aluminosilicates supporting a metal such as zinc, calcium silicate, hydrophobic zeolites (high silica zeolites), sepiolite, cancrinite, zeolites, and hydrated zirconium oxide; and metal-supporting porous substances such as silver-supporting zeolite, silver-supporting cancrinite, and vinylpyridine copolymers such as a silver-supporting porous styrene-divinylbenzene-vinylpyridine polymer and a silver-supporting porous divinylbenzene-vinylpyridine polymer. These porous particles can be used singly or in combination two or more thereof. Among these porous particles, it is preferable to use one or more selected from the group consisting of activated carbon, zinc oxide, aluminum oxide, silicon dioxide, hydrophobic zeolites, aluminosilicates, and metal-supporting aluminosilicates in view of effectiveness in the deodorization of causative agents of a breast cancerous skin ulcer disease odor including mainly acetoin especially.

The porous particles have a BET specific surface area of preferably 50 m²/g or more, more preferably 100 m²/g or more, and more preferably 200 m²/g or more in view of still more remarkably deodorizing causative agents of a breast cancerous skin ulcer disease odor including mainly acetoin. Although the upper limit thereof is not particularly limited, the BET specific surface area is practically 2000 m²/g or less. In addition, the average pore size of the porous particles is preferably 1 nm or more, preferably 50 nm or less, and more preferably 30 nm or less. The BET specific surface area is measured by a multipoint method using liquid nitrogen with a specific surface area and pore distribution measuring device manufactured by SHIMADZU CORPORATION "ASAP2020", and the value can be derived as long as the parameter C is positive. A mercury intrusion method can be used for measuring pore distribution.

The deodorant article including the sheet-like covering having the above-mentioned configuration is used for the deodorization of a breast cancerous skin ulcer disease odor emitted from a breast cancerous skin ulcer site and including mainly acetoin. For example, the deodorant article is disposed on the body surface of a wearer so that the longitudinal direction thereof is perpendicular to the median line of the wearer. In this case, it is preferable to dispose the deodorant article so that the deodorant article covers the mamma and passes the axilla with a tip thereof reaching the back side to cover a breast cancerous skin ulcer site with the deodorant article. Thus, a breast cancerous skin ulcer disease odor emitted from a breast cancerous skin ulcer site and including mainly acetoin can be effectively deodorized with the deodorant article. When a skin ulcer site on head and neck accompanies a skin ulcer site of the mamma, the deodorant article can also be used by wearing the deodorant article on the area from the mamma to the neck aslant. For example, a chest strap or adhesive tape may be used to fix the deodorant article to the body of a wearer.

Although the present invention has been described above based on preferred embodiments thereof, the present invention is not limited to the embodiments. For example, although the sheet-like covering used in the present invention has the topsheet, the absorbent core and the backsheet as basic members, the sheet-like covering may include other members.

In the present invention, although the polyhydroxyamine compound or the salt thereof and the porous particles are used as essential components as the deodorizer, the sheet-like covering used in the present invention may contain deodorant components other than these.

In connection with the above-mentioned embodiments, the present invention further discloses the following deodorizing methods and deodorant articles.
<1> A method for deodorizing a breast cancerous skin ulcer disease odor emitted from a breast cancerous skin ulcer site and including mainly acetoin using a deodorant article including a sheet-like covering,
   wherein the sheet-like covering contains a polyhydroxyamine compound or a salt thereof and porous particles,
   a wound contact surface in the sheet-like covering includes an air-through nonwoven fabric, and
   the sheet-like covering has a value of a ratio of an area S (cm²) to a thickness T (cm), S/T, of 300 or more and 3000 or less.
<2> The method as set forth in clause <1>, wherein the polyhydroxyamine compound or the salt thereof is tris(hydroxymethyl)aminomethane.
<3> The method as set forth in clause <1> or <2>, wherein the amount of the polyhydroxyamine compound and the salt thereof contained in the sheet-like covering is 0.0005 g/sheet or more and 0.05 g/sheet or less.
<4> The method as set forth in any one of clauses <1> to <3>, wherein the porous particles are one or more selected from the group consisting of activated carbon, zinc oxide, aluminum oxide, silicon dioxide, hydrophobic zeolites, aluminosilicates, and metal-supporting aluminosilicates.
<5> The method as set forth in any one of clauses <1> to <4>, wherein a content of the porous particles is 0.005 g/m² or more and 5.0 g/m² or less.
<6> The method as set forth in any one of clauses <1> to <5>,
   wherein the sheet-like covering has a liquid permeable topsheet used as the wound contact surface, a liquid-impermeable or sparingly-liquid-permeable backsheet, and a liquid-retentive absorbent core disposed therebetween, and
   one or more of the polyhydroxyamine compound or the salt thereof and the porous particles are contained in a part other than the wound contact surface in the topsheet in the sheet-like covering.
<7> The method as set forth in clause <6>, wherein one or more of the polyhydroxyamine compound or the salt thereof and the porous particles are contained between the topsheet and the absorbent core or contained between the backsheet and the absorbent core.
<8> The method as set forth in clause <7>,
   wherein a liquid permeable core-wrap sheet is disposed on a surface opposite to the topsheet or a surface opposite to the backsheet in the absorbent core, and
   one or more of the polyhydroxyamine compound or the salt thereof and the porous particles are contained in the core-wrap sheet.
<9> The method as set forth in clause <8>, wherein the core-wrap sheets are disposed on the surface opposite to the topsheet and the surface opposite to the backsheet in the absorbent core.
<10> The method as set forth in any one of clauses <1> to <9>, wherein a thickness of the absorbent core is 2 mm or more and 15 mm or less.
<11> A deodorant article including a sheet-like covering,
   wherein the deodorant article is disposed at a breast cancerous skin ulcer site and used for deodorizing a breast cancerous skin ulcer disease odor emitted from the site and including mainly acetoin,
   the sheet-like covering contains a polyhydroxyamine compound or a salt thereof and porous particles,
   a wound contact surface in the sheet-like covering includes an air-through nonwoven fabric, and
   the sheet-like covering has a value of a ratio of an area S (cm²) to a thickness T (cm), S/T, of 300 or more and 3000 or less.
<12> A method for manufacturing the deodorant article as set forth in clause <11>, the method comprising:
   a step of spraying an aqueous dispersion containing the polyhydroxyamine compound or the salt thereof or the porous particles on any fiber material for forming the deodorant article to adhere the polyhydroxyamine compound or the salt thereof or the porous particles thereto.
<13> Use of a sheet-like covering for deodorizing a breast cancerous skin ulcer disease odor emitted from a breast cancerous skin ulcer site and including mainly acetoin,
   wherein the sheet-like covering contains a polyhydroxyamine compound or a salt thereof and porous particles,
   a wound contact surface in the sheet-like covering includes an air-through nonwoven fabric, and
   the sheet-like covering has a value of a ratio of an area S (cm²) to a thickness T (cm), S/T, of 300 or more and 3000 or less.

### Examples

The present invention will be described by way of Examples further specifically hereinafter. However, the scope of the present invention is not limited to these Examples. Unless otherwise specified, "%" means "% by mass".

### [Example 1]

### (1) Manufacturing of absorbent core

A rectangular mixed stack of fibers including flap pulp and a water-absorbent polymer was manufactured, this mixed stack of fibers was cut to 15 × 20 cm, and the resultant was used as an absorbent core. The basis weight of the flap pulp in the absorbent core was 200 g/m², and the basis weight of the water-absorbent polymer was 35 g/m².

### (2) Provision of polyhydroxyamine compound or salt thereof

As the polyhydroxyamine compound, tris(hydroxymethyl)aminomethane (hereinafter also called merely "tris") was used. Tris was dissolved in water to prepare an aqueous solution. Citric acid was added to this aqueous solution. The mass ratio of tris to citric acid was 1:0.44. The pH of the aqueous solution was 7.5 at 25°C. This solution was incorporated into thin paper disposed on the surface opposite to the topsheet in the absorbent core.

### (3) Provision of porous particles

Activated carbon having a BET specific surface area of around 750 m²/g and a pore size of around 4.5 nm (activated with zinc chloride, SA1000, manufactured by FUTAMURA CHEMICAL CO., LTD.) was used as porous particles.

### (4) Provision of core-wrap sheet for top surface side

Thin paper having a basis weight of 16 g/m² was used as a core-wrap sheet. This thin paper was impregnated with the aqueous solution obtained in (2). The amount of tris impregnated was 0.5 g/m², and the amount of citric acid impregnated was 0.22g/m². The mass of tris was 0.015 g.

### (5) Provision of core-wrap sheet for back surface side

Coniferous kraft pulp to which porous particles provided in (3) was added was used as a raw material, and wet sheet making was performed to prepare thin paper having a basis weight of 16.5 g/m². The basis weight of the porous particles in the obtained core-wrap sheet was 0.5 g/m².

### (6) Provision of topsheet and backsheet

As a topsheet, an air-through nonwoven fabric including a sheath-core type composite fiber was used, the composite fiber having cores including polyethylene terephthalate and sheaths including polyethylene and having a fineness of 2 dtex. This air-through nonwoven fabric had a basis weight of 20 g/m², a density under a load of 2.5 g/cm² of 5.0 × 10⁻² g/cm³, and a thickness under the same load of 0.4 mm. As a backsheet, a polyethylene film having a basis weight of 19 g/m² was used.

### (7) Manufacturing of deodorant article including sheet-like covering

The core-wrap sheet for the top surface side and the core-wrap sheet for the back surface side were disposed on the surfaces of the absorbent core to obtain an absorbent member. This absorbent member was placed on the backsheet, and the top thereof was covered with the topsheet. The topsheet and the backsheet were joined in the portion which extended from the periphery of the absorbent member to obtain the deodorant article including a rectangular sheet-like covering. In this deodorant article, the thickness T was 0.7 cm, the area S was 300 cm², the length L was 20 cm, and the width W was 15 cm. The S/T was 429.

### [Example 2]

As porous particles, a zinc-supporting aluminosilicate having a BET specific surface area of around 250 m²/g and a pore size of around 20 nm (MIZUKANITE HP, manufactured by MIZUSAWA INDUSTRIAL CHEMICALS,LTD.) was used. These porous particles were supported on the core-wrap sheet. The basis weight of porous particles was made into 0.5 g/m². A deodorant article was obtained in the same manner as in Example 1 except the above.

### [Example 3]

In a core-wrap sheet for the top surface side used in Example 1, the basis weight of tris was changed to 0.05 g/m², and the mass of tris was changed to 0.00075 g (0.75 mg). As porous particles, a zinc-supporting aluminosilicate having a BET specific surface area of around 250 m²/g and a pore size of around 20 nm (MIZUKANITE HP, manufactured by MIZUSAWA INDUSTRIAL CHEMICALS,LTD.) was used. These porous particles were supported on the core-wrap sheet. The basis weight of the porous particles was 0.5 g/m². A deodorant article was obtained in the same manner as in Example 1 except these.

### [Example 4]

A hydrophobic zeolite having a BET specific surface area of 430 m²/g and a pore size of 1 nm (ABSCENTS 3000, manufactured by UNION SHOWA K.K.) was used. A deodorant article was obtained in the same manner as in Example 1 except these.

### [Comparative Example 1]

In Example 1, tris was not incorporated into the core-wrap sheet for the top surface side, and activated carbon was not incorporated into the core-wrap sheet for the back surface side. A deodorant article was obtained in the same manner as in Example 1 except these.

### [Comparative Example 2]

In Example 1, activated carbon was not incorporated into the core-wrap sheet for the back surface side. A deodorant article was obtained in the same manner as in Example 1 except this.

### [Comparative Example 3]

In Example 1, tris was not incorporated into the core-wrap sheet for the top surface side. A deodorant article was obtained in the same manner as in Example 1 except this.

### [Comparative Example 4]

In Example 2, tris was not incorporated into the core-wrap sheet for the top surface side. A deodorant article was obtained in the same manner as in Example 1 except this.

### [Comparative Example 5]

In Example 4, tris was not incorporated into the core-wrap sheet for the top surface side. A deodorant article was obtained in the same manner as in Example 1 except this.

### [Comparative Example 6]

In Example 1, tris was not incorporated into the core-wrap sheet for the top surface side. Green tea extract (FS500M) was incorporated into the core-wrap sheet for the back surface side instead of incorporating activated carbon. A deodorant article was obtained in the same manner as in Example 1 except these.

### [Comparative Example 7]

In Example 1, green tea extract (FS-500M, extract content 20%, produced by SHIRAIMATSU PHARMACEUTICAL Co., LTD.) was incorporated (prepared in terms of the extract content) instead of incorporating tris into the core-wrap sheet for the top surface side. Activated carbon was not incorporated into the core-wrap sheet for the back surface side. A deodorant article was obtained in the same manner as in Example 1 except these.

### [Comparative Example 8]

In Comparative Example 7, activated carbon was incorporated into the core-wrap sheet for the back surface side. This activated carbon is the same as the activated carbon used in Example 1. A deodorant article was obtained in the same manner as in Example 1 except this.

### [Comparative Example 9]

In Comparative Example 7, a zinc-supporting aluminosilicate (neutral) was incorporated into the core-wrap sheet for the back surface side. This zinc-supporting aluminosilicate is the same as the zinc-supporting aluminosilicate used in Example 2. A deodorant article was obtained in the same manner as in Example 1 except this.

### [Comparative Example 10]

In Comparative Example 7, a hydrophobic zeolite was incorporated into the core-wrap sheet for the back surface side. This hydrophobic zeolite is the same as the hydrophobic zeolite used in Example 4. A deodorant article was obtained in the same manner as in Example 1 except this.

### [Evaluation]

The deodorant articles obtained in Examples and Comparative Examples were evaluated by the following method. The following Tables 2 and 3 show the results.

### [Evaluation 1]

(a1) Acetoin was diluted with propylene glycol (PG) to an acetoin concentration of 0.1% to prepare a solution of an offensive odor substance. Then, 2 ml of the solution was used, and this was dropped at the center of the topsheet in the deodorant article of each of Examples and Comparative Examples.

(a2) Separately from this operation, a pseudo-breast cancer offensive odor composition shown in the following Table 1 and containing acetoin was prepared, and this composition was 10-fold diluted with propylene glycol to prepare a solution of offensive odor substances. Then, 2 ml of the solution was used, and this was dropped at the center of the topsheet in the deodorant article of each of Examples and Comparative Examples.

**[Table 1]**

| Component | Content (g) |
|---|---|
| Normal butyric acid | 0.2 |
| Isovaleric acid | 0.1 |
| Acetoin | 0.0001 |
| Dimethyl trisulfide | 0.0001 |
| Phenol | 0.01 |
| Propylene glycol | Balance |
| Total | 15.0 |

The following evaluations of (b1) and (b2) were performed as to each of the solutions of offensive odor substances of the (a1) and the (a2).

(b1) Method in which offensive odor which leaks outside clothes when a deodorant article was worn on skin with clothes worn thereover ("leak of offensive odor") was assumed

The deodorant article was laid with the surface of the deodorant article on which the solution of an offensive odor substance was dropped down, the periphery was fixed with 5 cm-wide adhesive tapes (P-cut tape, manufactured by TERAOKA SEISAKUSHO CO.,LTD.), and the offensive odor was evaluated 5 cm thereabove.

(b2) Method in which offensive odor which rises from area between clothes and skin to bosom when a deodorant article was worn on skin ("rising of offensive odor") was assumed

The deodorant article was disposed vertically, and the offensive odor was evaluated 5 cm thereabove.

In (b1) and (b2), the following scale of 0 to 5, which is in accordance with the scale used in the offensive odor control law of the Ministry of the Environment, was used for the measure of the intensity of an offensive odor.
0: No odor
1: Odor which can be perceived barely
2: So weak odor that it is found what odor the odor is
3: Odor which can be perceived easily
4: Strong odor
5: Overpowering odor

**[Table 2]**

| | | Example 1 | Example 2 | Example 3 | Example 4 |
|---|---|---|---|---|---|
| Deodorant article | Topsheet | Air-through nonwoven fabric | | | |
| | Thickness T(cm) | 0.7 | 0.7 | 0.7 | 0.7 |
| | Area S(cm²) | 300 | 300 | 300 | 300 |
| | S/T | 429 | 429 | 429 | 429 |
| | Length L(cm) | 20 | 20 | 20 | 20 |
| | Width W(cm) | 15 | 15 | 15 | 15 |
| Core-wrap sheet on top surface side | Type | Tris/Citric acid | Tris/Citric acid | Tris/Citric acid | Tris/Citric acid |
| | Basis weight (g/m²) | 0.5 | 0.5 | 0.05 | 0.5 |
| | Mass (g/sheet) | 0.015 | 0.015 | 0.0015 | 0.015 |
| Core-wrap sheet on back surface side | Type | Activated carbon | Zinc-supporting aluminosilicate | Zinc-supporting aluminosilicate | Hydrophobic zeolite |
| | BET specific surface area (m²/g) | 750 | 250 | 250 | 430 |
| | Pore size (nm) | 4.5 | 20 | 20 | 1 |
| | Basis weight (g/m²) | 0.5 | 0.5 | 0.5 | 0.5 |
| Evaluation of performance (on acetoin) | Leak of offensive odor | 1.5 | 1.5 | 2 | 1.5 |
| | Rising of offensive odor | 2 | 2 | 2 | 2 |
| Evaluation of performance (on pseudo-breast cancer odor) | Leak of offensive odor | 1.5 | 1.5 | 2 | 1.5 |
| | Rising of offensive odor | 2 | 2 | 2 | 2 |

**[Table 3]**

| | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|
| Deodorant article | Topsheet | Air-through nonwoven fabric | | | | | | | | | |
| | Thickness T(cm) | 0.7 | 0.7 | 0.7 | 0.7 | 0.7 | 0.7 | 0.7 | 0.7 | 0.7 | 0.7 |
| | Area S(cm²) | 300 | 300 | 300 | 300 | 300 | 300 | 300 | 300 | 300 | 300 |
| | S/T | 429 | 429 | 429 | 429 | 429 | 429 | 429 | 429 | 429 | 429 |
| | Length L(cm) | 20 | 20 | 20 | 20 | 20 | 20 | 20 | 20 | 20 | 20 |
| | Width W(cm) | 15 | 15 | 15 | 15 | 15 | 15 | 15 | 15 | 15 | 15 |
| Core-wrap sheet on top surface side | Type | - | Tris/Citric acid | - | - | - | - | Green tea extract | | | |
| | Basis weight (g/m²) | - | 0.5 | - | - | - | - | 0.5 | 0.5 | 0.5 | 0.5 |
| | Mass (g/sheet) | - | 0.015 | - | - | - | - | 0.015 | 0.015 | 0.015 | 0.015 |
| Core-wrap sheet on back surface side | Type | - | - | Activated carbon | Zinc-supporting aluminosilicate | Hydrophobic zeolite | Green tea extract | - | Activated carbon | Zinc-supporting aluminosilicate | Hydrophobic zeolite |
| | BET specific surface area (m²/g) | - | - | 750 | 250 | 430 | - | - | 750 | 250 | 430 |
| | Pore size (nm) | - | - | 4.5 | 20 | 1 | - | - | 4.5 | 20 | 1 |
| | Basis weight (g/m²) | - | - | 0.5 | 0.5 | 0.5 | 0.5 | - | 0.5 | 0.5 | 0.5 |
| Evaluation of performance (on acetoin) | Leak of offensive odor | 4 | 3 | 3 | 3 | 3 | 3 | 4 | 3 | 3 | 3 |
| | Rising of offensive odor | 4 | 3 | 4 | 3 | 3 | 4 | 4 | 4 | 4 | 4 |
| Evaluation of performance (on pseudo-breast cancer odor) | Leak of offensive odor | 4 | 3 | 3 | 3 | 3 | 4 | 4 | 3 | 3 | 3 |
| | Rising of offensive odor | 4 | 3 | 4 | 4 | 4 | 4 | 4 | 4 | 4 | 4 |

As shown in Tables 2 and 3, it was found that the deodorant articles of Examples achieved improvement in evaluations of the leak of an offensive odor, namely effective prevention of the leak of the offensive odor which leaked from clothes to the outside. In contrast, it was found that the deodorant articles of the Comparative Examples exhibited insufficient effects of preventing the leak of an offensive odor.

As shown in Tables 2 and 3, it was found that the deodorant articles of the Examples achieved improvement in evaluations of the rising of an offensive odor, namely effective prevention of the offensive odor from rising from the bosom. In contrast, it was found that the deodorant articles of the Comparative Examples exhibited insufficient effects of preventing the rising of an offensive odor.

### [Example 5]

The deodorant article shown in Fig. 1 was manufactured instead of the deodorant article manufactured in Example 2. First, 0.05 g/cm² of tris was supported on the core-wrap sheet to be disposed on the top surface side. Then, 0.5 g/cm² of a zinc-supporting aluminosilicate was supported on the core-wrap sheet to be disposed on the back surface side. The deodorant article measured 41 cm in the length L, 23 cm in the width W, 727 cm² in the area S, and 0.6 cm in the thickness T, and S/T = 1212.

### [Example 6]

A rectangular deodorant article measuring 0.7 cm in the thickness T, 30 cm in the length L, 14 cm in the width W, and 420 cm² in the area S was manufactured using the core-wrap sheets manufactured in Example 5. The S/T was 600. The same procedure as in Example 5 was performed except this.

### [Comparative Example 11]

A rectangular deodorant article wherein the deodorant article measured 1.6 cm in the thickness T, 14 cm in the length L, 14 cm in the width W, and 196 cm² in the area S, and S/T = 123 was manufactured using the core-wrap sheets manufactured in Example 5.

### [Evaluation 2]

Skin ulcer sites of breast cancer were assumed, and specifically, the absorbent cotton pieces impregnated with 1 g of the above-mentioned solution of the offensive odor substances of (a2) were fixed at a total of three points (two points on the top of the mamma and one point in the axillary site) on an upper half mannequin for men (assumed to be the breast after mastectomy; the chest measurement was around 80 cm). The deodorant article of each of Examples 5 and 6 and Comparative Example 11 was worn thereover using medical tape (Kind Removal Silicone Tape, manufactured by 3M Company), a T-shirt was further worn thereover, and the T-shirt was put on and taken off repeatedly 10 times.

Consequently, when the deodorant article of Example 5 or Example 6 was worn, the three places emitting an offensive smell can be covered with a single sheet of the article. Additionally, when the T-shirt was worn over the deodorant article, the dislocation of the deodorant article did not occur. The offensive odor from the whole mannequin was reduced. In contrast, in the case of the deodorant article of Comparative Example 11, when the T-shirt worn over the deodorant article was taken off, the dislocation of the deodorant article occurred, and the effect of reducing an offensive odor was insufficient.

### Industrial Applicability

As described in detail above, a breast cancerous skin ulcer disease odor can be reduced and deodorized more effectively than before according to the present invention.

## Claims

1. A method for deodorizing a breast cancerous skin ulcer disease odor emitted from a breast cancerous skin ulcer site and including mainly acetoin using a deodorant article comprising a sheet-like covering,
wherein the sheet-like covering comprises a polyhydroxyamine compound or a salt thereof and porous particles,
a wound contact surface in the sheet-like covering comprises an air-through nonwoven fabric, and
the sheet-like covering has a value of a ratio of an area S (cm²) to a thickness T (cm), S/T, of 300 or more and 3000 or less.

2. The method according to claim 1, wherein the polyhydroxyamine compound or the salt thereof is tris(hydroxymethyl)aminomethane.

3. The method according to claim 1 or 2, wherein the amount of the polyhydroxyamine compound and the salt thereof contained in the sheet-like covering is 0.0005 g/sheet or more and 0.05 g/sheet or less.

4. The method according to any one of claims 1 to 3, wherein the porous particles are one or more selected from the group consisting of activated carbon, zinc oxide, aluminum oxide, silicon dioxide, hydrophobic zeolites, aluminosilicates, and metal-supporting aluminosilicates.

5. The method according to any one of claims 1 to 4, wherein a content of the porous particles is 0.005 g/m² or more and 5.0 g/m² or less.

6. The method according to any one of claims 1 to 5,
wherein the sheet-like covering has a liquid permeable topsheet used as the wound contact surface, a liquid-impermeable or sparingly-liquid-permeable backsheet, and a liquid-retentive absorbent core disposed therebetween, and
one or more of the polyhydroxyamine compound or the salt thereof and the porous particles are contained in a part other than the wound contact surface in the topsheet in the sheet-like covering.

7. The method according to claim 6, wherein one or more of the polyhydroxyamine compound or the salt thereof and the porous particles are contained between the topsheet and the absorbent core or contained between the backsheet and the absorbent core.

8. The method according to claim 7,
wherein a liquid permeable core-wrap sheet is disposed on a surface opposite to the topsheet or a surface opposite to the backsheet in the absorbent core, and
one or more of the polyhydroxyamine compound or the salt thereof and the porous particles are contained in the core-wrap sheet.

9. The method according to claim 8, wherein the core-wrap sheets are disposed on the surface opposite to the topsheet and the surface opposite to the backsheet in the absorbent core.

10. The method according to any one of claims 1 to 9, wherein a thickness of the absorbent core is 2 mm or more and 15 mm or less.

11. A deodorant article comprising a sheet-like covering,
wherein the deodorant article is disposed at a breast cancerous skin ulcer site and used for deodorizing a breast cancerous skin ulcer disease odor emitted from the site and including mainly acetoin, and
the sheet-like covering comprises a polyhydroxyamine compound or a salt thereof and porous particles,
a wound contact surface in the sheet-like covering comprises an air-through nonwoven fabric, and
the sheet-like covering has a value of a ratio of an area S (cm²) to a thickness T (cm), S/T, of 300 or more and 3000 or less.

12. A method for manufacturing the deodorant article according to claim 11, the method comprising:
a step of spraying an aqueous dispersion comprising the polyhydroxyamine compound or the salt thereof or the porous particles on any fiber material for forming the deodorant article to adhere the polyhydroxyamine compound or the salt thereof or the porous particles thereto.

13. Use of a sheet-like covering for deodorizing a breast cancerous skin ulcer disease odor emitted from a breast cancerous skin ulcer site and including mainly acetoin,
wherein the sheet-like covering comprises a polyhydroxyamine compound or a salt thereof and porous particles,
a wound contact surface in the sheet-like covering comprises an air-through nonwoven fabric, and
the sheet-like covering has a value of a ratio of an area S (cm²) to a thickness T (cm), S/T, of 300 or more and 3000 or less.

## Patentansprüche

1. Verfahren zum Desodorieren eines Brustkrebs-Hautgeschwür-Krankheitsgeruchs, der von einer Brustkrebs-Hautgeschwür-Stelle emittiert wird und hauptsächlich Acetoin einschließt, unter Verwendung eines Deodorantartikels, der eine folienartige Abdeckung umfasst,
wobei die folienartige Abdeckung eine Polyhydroxyamin-Verbindung oder ein Salz davon und poröse Teilchen umfasst,
eine Wundkontaktfläche in der folienartigen Abdeckung einen luftdurchlässigen Vliesstoff umfasst, und
die folienartige Abdeckung einen Wert des Verhältnisses einer Fläche S (cm²) zu einer Dicke T (cm), S/T, von 300 oder mehr und 3000 oder weniger aufweist.

2. Verfahren gemäß Anspruch 1, wobei die Polyhydroxyamin-verbindung oder das Salz davon Tris(hydroxymethyl)aminomethan ist.

3. Verfahren gemäß Anspruch 1 oder 2, wobei die Menge der Polyhydroxyamin-Verbindung und des Salzes davon, die in der folienartigen Abdeckung enthalten ist, 0,0005 g/Folie oder mehr und 0,05 g/Folie oder weniger beträgt.

4. Verfahren gemäß mindestens einem der Ansprüche 1 bis 3, wobei die porösen Teilchen eines oder mehrere sind, die ausgewählt sind aus einer Gruppe bestehend aus Aktivkohle, Zinkoxid, Aluminiumoxid, Siliciumdioxid, hydrophoben Zeolithen, Aluminosilicaten und metalltragenden Aluminosilicaten.

5. Verfahren gemäß mindestens einem der Ansprüche 1 bis 4, wobei der Gehalt der porösen Teilchen 0,005 g/m² oder mehr und 5,0 g/m² oder weniger beträgt.

6. Verfahren gemäß mindestens einem der Ansprüche 1 bis 5,
wobei die folienartige Abdeckung eine flüssigkeitsdurchlässige Oberschicht, die als Wundkontaktfläche verwendet wird, eine flüssigkeitsundurchlässige oder nur wenig flüssigkeitsdurchlässige Unterschicht und einen dazwischen angeordneten flüssigkeitsrückhaltenden absorbierenden Kern aufweist, und
eine oder mehrere der Polyhydroxyamin-Verbindungen oder des Salzes davon und die porösen Teilchen in einem anderen Teil als der Wundkontaktfläche in der Oberschicht in der folienartigen Abdeckung enthalten sind.

7. Verfahren gemäß Anspruch 6, wobei eine oder mehrere der Polyhydroxyamin-Verbindungen oder des Salzes davon und der porösen Teilchen zwischen der Oberschicht und dem absorbierenden Kern oder zwischen der Unterschicht und dem absorbierenden Kern enthalten sind.

8. Verfahren gemäß Anspruch 7,
wobei in dem absorbierenden Kern eine flüssigkeitsdurchlässige Kernumwicklungsschicht auf einer Oberfläche gegenüber der Oberschicht oder einer Oberfläche gegenüber der Unterschicht angeordnet ist, und
eine oder mehrere der Polyhydroxyamin-Verbindungen oder des Salzes davon und der porösen Teilchen in der Kernumwicklungsschicht enthalten sind.

9. Verfahren gemäß Anspruch 8, wobei in dem absorbierenden Kern die Kernumwicklungsschichten auf der Oberfläche gegenüber der Oberschicht und der Oberfläche gegenüber der Unterschicht angeordnet sind.

10. Verfahren gemäß mindestens einem der Ansprüche 1 bis 9, wobei die Dicke des absorbierenden Kerns 2 mm oder mehr und 15 mm oder weniger beträgt.

11. Deodorant-Artikel, umfassend eine folienartige Abdeckung,
wobei der Deodorant-Artikel für eine Brustkrebs-Hautgeschwür-Stelle vorgesehen ist und zum Desodorieren eines von der Stelle emittierten Brustkrebs-Hautgeschwür-Krankheitsgeruchs verwendet wird und hauptsächlich Acetoin einschließt, und
die folienartige Abdeckung eine Polyhydroxyamin-Verbindung oder ein Salz davon und poröse Teilchen umfasst,
eine Wundkontaktfläche in der folienartigen Abdeckung einen luftdurchlässigen Vliesstoff umfasst, und
die folienartige Abdeckung einen Wert des Verhältnisses einer Fläche S (cm²) zu einer Dicke T (cm), S/T, von 300 oder mehr und 3000 oder weniger aufweist.

12. Verfahren zur Herstellung des Deodorant-Artikels gemäß Anspruch 11, wobei das Verfahren umfasst:
einen Schritt, bei dem eine wässrige Dispersion, die die Polyhydroxyamin-Verbindung oder das Salz davon oder die porösen Teilchen enthält, auf ein beliebiges Fasermaterial zum Bilden des Deodorant-Artikels aufgesprüht wird, um die Polyhydroxyamin-Verbindung oder das Salz davon oder die porösen Teilchen daran zu haften.

13. Verwendung einer folienartigen Abdeckung zum Desodorieren eines Brustkrebs-Hautgeschwür-Krankheitsgeruchs, der von einer Brustkrebs-Hautgeschwür-Stelle emittiert wird und hauptsächlich Acetoin einschließt,
wobei die folienartige Abdeckung eine Polyhydroxyamin-Verbindung oder ein Salz davon und poröse Teilchen umfasst,
eine Wundkontaktfläche in der folienartigen Abdeckung einen luftdurchlässigen Vliesstoff umfasst, und
die folienartige Abdeckung einen Wert des Verhältnisses einer Fläche S (cm²) zu einer Dicke T (cm), S/T, von 300 oder mehr und 3000 oder weniger aufweist.

## Revendications

1. Procédé de désodorisation d'une odeur de maladie d'ulcère cutané cancéreux du sein émise à partir d'un site d'ulcère cutané cancéreux du sein et incluant principalement de l'acétoïne utilisant un article déodorant comprenant un revêtement de type feuille,
dans lequel le revêtement de type feuille comprend un composé polyhydroxyamine ou un sel de celui-ci et des particules poreuses,
une surface de contact avec la plaie dans le revêtement de type feuille comprenant un tissu non tissé perméable à l'air, et
le revêtement de type feuille présentant une valeur d'un rapport d'une superficie S (cm²) sur une épaisseur T (cm), S/T, de 300 ou plus et de 3000 ou moins.

2. Procédé selon la revendication 1, dans lequel le composé polyhydroxyamine ou le sel de celui-ci est du tris(hydroxyméthyl)aminométhane.

3. Procédé selon la revendication 1 ou la revendication 2, dans lequel la quantité du composé polyhydroxyamine et du sel de celui-ci contenu dans le revêtement de type feuille est de 0,0005 g/feuille ou plus et 0,05 g/feuille ou moins.

4. Procédé selon l'une quelconque des revendications 1 à 3, dans lequel les particules poreuses sont une ou plusieurs choisies dans le groupe consistant en le charbon actif, l'oxyde de zinc, l'oxyde d'aluminium, le dioxyde de silicium, les zéolites hydrophobes, les aluminosilicates et les aluminosilicates porteurs de métal.

5. Procédé selon l'une quelconque des revendications 1 à 4, dans lequel une teneur des particules poreuses est de 0,005 g/m² ou plus et de 5,0 g/m² ou moins.

6. Procédé selon l'une quelconque des revendications 1 à 5,
dans lequel le revêtement de type feuille présente une feuille supérieure perméable aux liquides utilisée comme surface de contact avec la plaie, une feuille arrière imperméable aux liquides ou peu perméable aux liquides, et un noyau absorbant à rétention de liquide disposé entre ceux-ci, et
un ou plusieurs du composé polyhydroxyamine ou du sel de celui-ci et des particules poreuses sont contenus dans une partie autre que la surface de contact avec la plaie dans la feuille supérieure dans le revêtement de type feuille.

7. Procédé selon la revendication 6, dans lequel un ou plusieurs du composé polyhydroxyamine ou du sel de celui-ci et des particules poreuses sont contenus entre la feuille supérieure et le noyau absorbant ou contenus entre la feuille arrière et le noyau absorbant.

8. Procédé selon la revendication 7,
dans lequel une feuille d'enveloppe de noyau perméable aux liquides est disposée sur une surface opposée à la feuille supérieure ou une surface opposée à la feuille arrière dans le noyau absorbant, et
dans lequel un ou plusieurs du composé polyhydroxyamine ou du sel de celui-ci et des particules poreuses sont contenus dans la feuille d'enveloppe de noyau.

9. Procédé selon la revendication 8, dans lequel les feuilles d'enveloppe de noyau sont disposées sur la surface opposée à la feuille supérieure et la surface opposée à la feuille arrière dans le noyau absorbant.

10. Procédé selon l'une quelconque des revendications 1 à 9, dans lequel une épaisseur du noyau absorbant est 2 mm ou plus et 15 mm ou moins.

11. Article déodorant comprenant un revêtement de type feuille,
dans lequel l'article déodorant est disposé au niveau d'un site d'ulcère cutané cancéreux du sein et utilisé pour désodoriser une odeur de maladie d'ulcère cutané cancéreux du sein émise par le site et incluant principalement de l'acétoïne, et
le revêtement de type feuille comprenant un composé polyhydroxyamine ou un sel de celui-ci et des particules poreuses,
une surface de contact avec la plaie dans le revêtement de type feuille comprenant un tissu non tissé perméable à l'air, et
le revêtement de type feuille présentant une valeur d'un rapport d'une superficie S (cm²) sur une épaisseur T (cm), S/T, de 300 ou plus et 3000 ou moins.

12. Procédé de fabrication de l'article déodorant selon la revendication 11, le procédé comprenant :
une étape de pulvérisation d'une dispersion aqueuse comprenant le composé polyhydroxyamine ou le sel de celui-ci ou les particules poreuses sur tout matériau fibreux pour former l'article déodorant afin qu'elle adhère au composé polyhydroxyamine ou au sel de celui-ci ou aux particules poreuses de celui-ci.

13. Utilisation d'un revêtement de type feuille pour désodoriser une odeur de maladie d'ulcère cutané cancéreux du sein émise par un site d'ulcère cutané cancéreux du sein et incluant principalement de l'acétoïne,
dans lequel le revêtement de type feuille comprend un composé polyhydroxyamine ou un sel de celui-ci et des particules poreuses,
une surface de contact avec la plaie dans le revêtement de type feuille comprenant un tissu non tissé perméable à l'air, et
le revêtement de type feuille ayant une valeur d'un rapport d'une surface S (cm²) sur une épaisseur T (cm), S/T, de 300 ou plus et de 3000 ou moins.
